# EUROPEAN PATENT SPECIFICATION

(11) **EP 2 904 080 B1**
(45) Date of publication and mention of the grant of the patent: **12.12.2018**
(21) Application number: 13774143.5
(22) Date of filing: 07.10.2013
(51) Int. Cl.: C11B 9/00, A61L 9/01, A61Q 13/00, C07C 33/03, C07C 43/178, C07C 69/145, C11D 3/50, A61K 8/34, A61K 8/37, C07C 29/44, C07C 67/08, C07C 67/283, C07C 33/025, C07C 69/007, C07C 69/533, C07C 41/20, C07C 45/62, A23L 27/20

(54) **FLAVOR AND FRAGRANCE FORMULATION (I)**
GESCHMACKS- UND DUFTFORMULIERUNG (I)
FORMULATION (I) D'ARÔME ET DE PARFUM

(30) Priority: 08.10.2012 EP 12187637
(43) Date of publication of application: 12.08.2015
(73) Proprietor: DSM IP Assets B.V., 6411 TE Heerlen (NL)
(72) Inventor: BEUMER, Raphael, CH-4002 Basel (CH); TSCHUMI, Johannes, CH-4002 Basel (CH); GRESSLY, Michael, CH-4002 Basel (CH)
(74) Representative: Steck, Melanie
(86) International application number: PCT/EP2013/070836
(87) International publication number: WO 2014/056851

(56) References cited:
- EP-A1- 0 044 413
- JP-A- 2000 319 684
- US-A- 2 833 812

## Description

The present invention relates to the use of specific organic compounds as flavor and fragrance material. Furthermore the invention relates to new specific organic compounds, as well as to flavor and fragrance formulations comprising at least one of the specific organic compounds.

In the flavor and fragrance industry there is always a need and demand for compounds that enhance, modify, improve or otherwise positively influence an odor note and therefore give perfumers or other persons the ability to create new fragrances for perfumes, colognes, personal care products, household products or any other products, which comprise flavor and fragrance materials.

Surprisingly it was found that the compounds of formula (I) are very useful as flavor and fragrance materials.

Therefore the present invention is related to the use of a compound of formula (I) 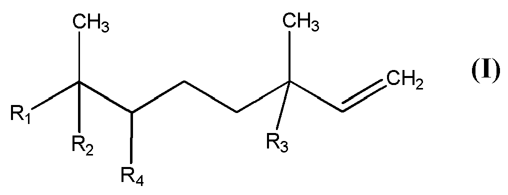 wherein
R₁ signifies - CH₃ (methyl), CH₂CH₃ (ethyl) or -CH₂CH₂CH₃ (n-propyl), and
R₂ signifies -H (hydrogen), and
R₃ signifies -O(CO)CH₃ (acetyloxy), and
R₄ signifies -H (hydrogen) or -CH₃ (methyl),
as flavor and fragrance material;
with the proviso that R¹ is **not** methyl, if R² **and** R⁴ signify hydrogen.

Preferred is the use of at least one compound selected from the group consisting of the compounds of formulae (Ia) - (Ic), (Ie) - (Ii) and any mixture thereof 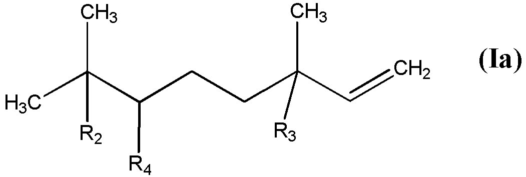 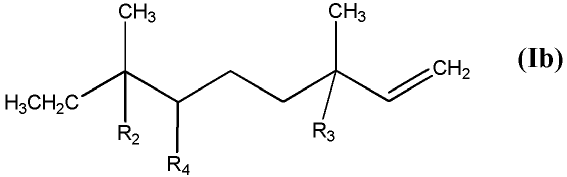 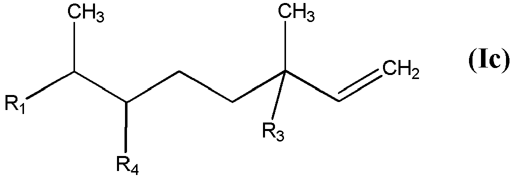 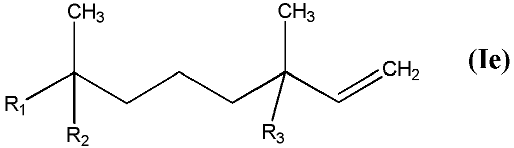 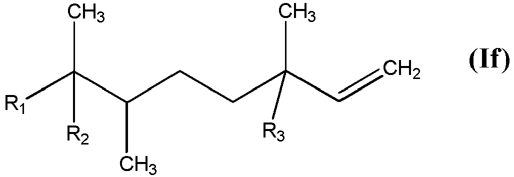 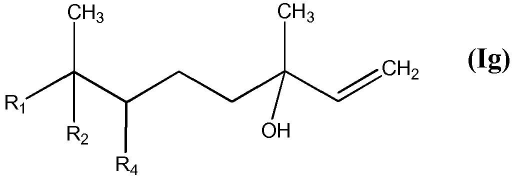 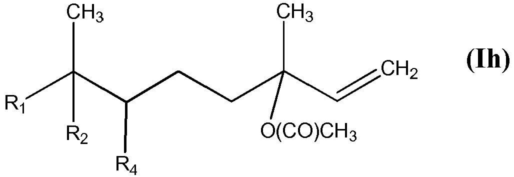 and 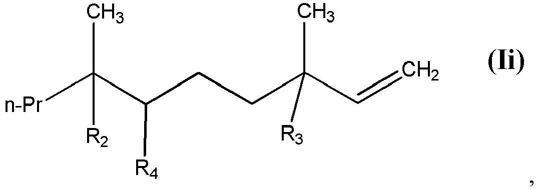 wherein R₁, R₂, R₃ and R₄ have the meanings as defined above,
as flavor and fragrance material.

Among these the use of a compound of formula (Ii) or of any mixture thereof is even more preferred.

More preferred is the use of at least one compound selected from the group consisting of compounds of formulae (II), (V) and (VII) and any mixture thereof

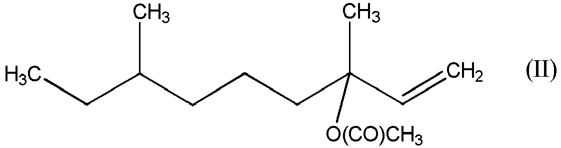

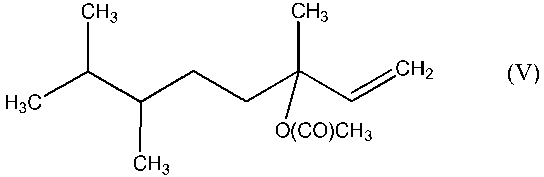

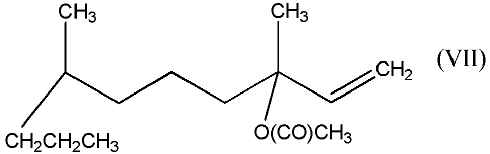

The compounds of formula (I) may be used as such or in combination with other compounds of formula (I) or other compounds which are known as flavor and fragrance material.

Such other compounds which are known as flavor and fragrance material include all known odorant molecules selected from the extensive range of natural products and synthetic molecules currently available, such as essential oils, alcohols, aldehydes and ketones, ethers and acetals, esters and lactones, macrocycles and heterocycles, and/or in admixture with one or more ingredients or excipients conventionally used in conjunction with odorants in flavor fragrance formulations, for example, carrier materials, and other auxiliary agents commonly used in the art.

The flavor and fragrance material of the present invention is used in a flavor and fragrance formulation.
Such a flavor and fragrance formulation comprises other ingredients.
The flavor and fragrance formulation according to the present invention can be in any form. Usually it is in a solid, gel-like or liquid (or a combination thereof) form. It can also be in an encapsulated form (i.e. a liquid formulation encapsulated by a suitable matrix material).

Therefore the present invention also relates to flavor and fragrance formulations comprising
(i) at least one compound of formula (I) 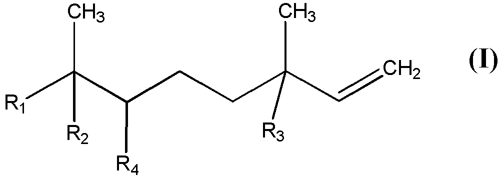 wherein
   R₁ signifies - CH₃, CH₂CH₃ or -CH₂CH₂CH₃, and
   R₂ signifies -H, and
   R₃ signifies -O(CO)CH₃, and
   R₄ signifies -H or -CH₃;
   with the proviso that R¹ is **not** methyl, if R² **and** R⁴ signify hydrogen;
   and with the preferences as given above.

Preferred are flavor and fragrance formulations comprising at least one compound selected from the group consisting of the compounds of formulae (Ia) - (Ic), (Ie) - (Ii) and their mixtures 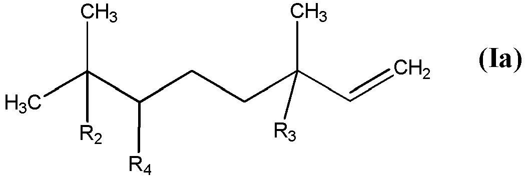 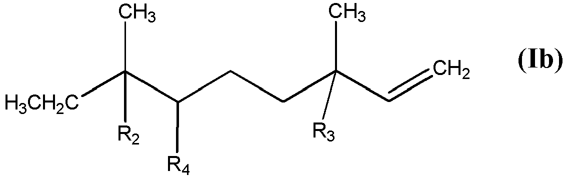 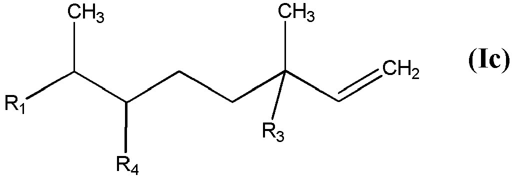 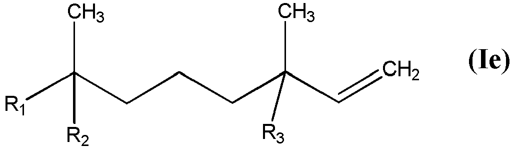 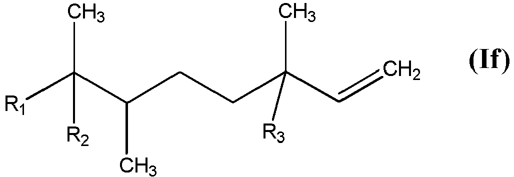 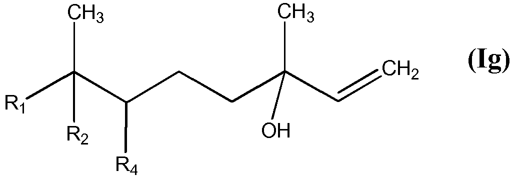 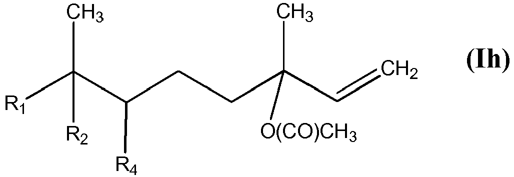 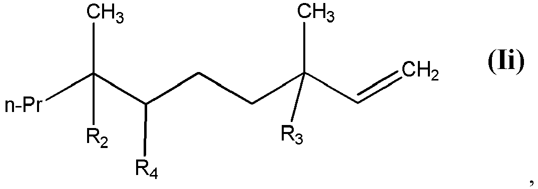 wherein R₁, R₂, R₃ and R₄ have the meanings as defined above, and wherein the compounds of formula (Id) and (Ii) are especially preferred.

More preferred are flavor and fragrance formulations comprising at least one compound selected from the group consisting of the compounds of formulae (II), (V) and (VII) and their mixtures

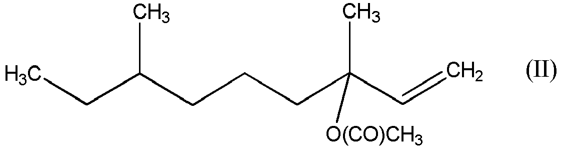

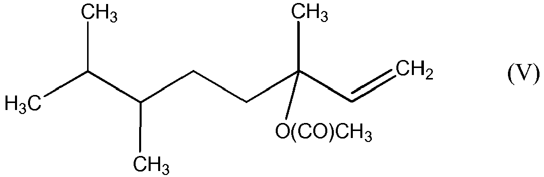

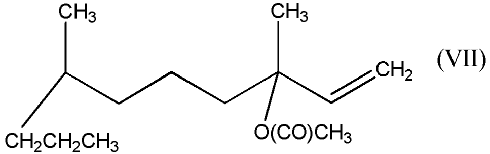

When a compound of formula (I) with the preferences as given above is used in a flavor and fragrance formulation, then the amount thereof is in the range of 0.0001 - 10 weight-% (wt-%), related to the total weight of the flavor and fragrance formulation. Preferably is an amount in the range of 0.01 - 5 wt-%, based on the total weight of the flavor and fragrance formulation.

Therefore the present invention relates to liquid flavor and fragrance formulations comprising
(i) 0.0001 - 10 wt-% (preferably 0.01 - 5 wt-%), related to the total weight of the flavor and fragrance formulation, of at least one compound of formula (I) with the preferences as given above.

The flavor and fragrance formulations according to the present invention can comprise further ingredients (= auxiliary compounds), such as any further perfuming compounds solvents, adjuvants, thickeners, surface active agents, pigments, extenders, rheology modifiers, dyestuffs, antioxidants, fillers and the like.

Many flavor and fragrance formulations are in a liquid form (like a perfume, cologne, etc.). Therefore, for such liquid formulation a (diluent) solvent is present. Such common diluents are i.e. dipropyleneglycol, isopropyl myristate, triethylcitrate and alcohols (such as ethanol).

Further examples of fine perfumery are Eau de perfume, Eau de Toilette, Eau de Cologne and Splash Cologne. Fine perfumery products are commonly based on an alcoholic solution as diluent. However fine perfumery products using an oil or wax as diluent are also included within the meaning of this invention. The compounds can be employed in widely varying amounts, depending upon the specific application and on the nature and quantity of other odourant ingredients.
When used in a (fine) perfume, the amount of the compound of formula (I) with the preferences as given above is usually between 0.01 - 10 wt-%, based on the total weight of the (fine) perfume.
However, these values and ranges are given only by way of example, since the experienced perfumer may also achieve effects or may create novel accords with lower or higher concentrations.

Furthermore the present invention relates to liquid flavor and fragrance formulations comprising
(i) at least one compound of formula (I) with the preferences as given above, and
(ii) at least one diluent chosen from the group consisting of dipropyleneglycol, isopropylmyristate, triethylcitrate and alcohols (such as ethanol), and optionally
(iii) at least one auxiliary compound selected from the group consisting of perfuming compounds solvents, adjuvants, thickeners, surface active agents, pigments, extenders, rheology modifiers, dyestuffs, antioxidants and fillers.

Furthermore the present invention relates to solid flavor and fragrance formulations comprising
(i) at least one compound of formula (I) with the preferences as given above and
(ii) at least one auxiliary compound selected from the group consisting of perfuming compounds solvents, adjuvants, thickeners, surface active agents, pigments, extenders, rheology modifiers, dyestuffs, antioxidants and fillers.

The compounds of formula (I) with the preferences as given above may be used in a broad range of flavor and fragrance formulations, e.g. in any field of fine and functional perfumery, such as perfumes, air care products, household products, laundry products, body care products and cosmetics.

The compounds as described hereinabove may be employed in a flavor and fragrance formulation simply by directly mixing at least one compound of formula (I) with the preferences as given above, a mixture thereof, or a fragrance composition with the other ingredients used in the final product, or they may, in an earlier step, be entrapped with an entrapment material, for example, polymers, capsules, microcapsules and nanocapsules, liposomes, film formers, absorbents such as carbon or zeolites, cyclic oligosaccharides and mixtures thereof, or they may be chemically bonded to substrates, which are adapted to release the fragrance molecule upon application of an external stimulus such as light, enzyme, or the like, and then mixed with the other ingredients used in the final product.

Thus, the invention additionally provides a method of manufacturing a flavor and fragrance formulation, comprising the incorporation of a compound of formula (I) with the preferences as given above, as a fragrance ingredient, either by directly admixing the compound to the other ingredients used in the final product or by admixing a fragrance composition comprising a compound of formula (I) with the preferences as given above, which may then be mixed with the other ingredients used in the final product, using conventional techniques and methods.

Through the addition of an olfactory acceptable amount of a compound of the present invention as hereinabove described, or a mixture thereof, the odor notes of a consumer product base will be improved, enhanced or modified.

Thus, the invention furthermore provides a method for improving, enhancing or modifying a consumer product (= final product) base by means of the addition thereto of an olfactory acceptable amount of a compound of formula (I) with the preferences as given above, or a mixture thereof.

In the context of the present invention the olfactory effective amount is to be understood as the amount of the at least one compound of formula (I) with the preferences as given above in a flavor and fragrance formulation will contribute to its particular olfactory characteristics, but the olfactory effect of the flavor and fragrance formulation will be the sum of the effects of each of the perfumes or fragrance ingredients. Thus the compounds of the invention can be used to alter the aroma characteristics of the flavor and fragrance formulation, or by modifying the olfactory reaction contributed by another ingredient in the composition. The amount will vary depending on many factors including other ingredients, their relative amounts and the effect that is desired.

As used herein, "consumer product (= final product)" means a composition for use as a consumer product to fulfill specific actions, such as cleaning, softening, and caring or the like. Examples of such products include fine perfumery, e.g. perfume and eau de toilette; fabric care, household products and personal care products such as laundry care detergents, rinse conditioner, personal cleansing composition, detergent for dishwasher, surface cleaner; laundry products, e.g. softener, bleach, detergent; body care products, e.g. shampoo, shower gel; air care products and cosmetics, e.g. deodorant, vanishing creme. This list of products is given by way of illustration and is not to be regarded as being in any way limiting.

The compounds of formula (I) may be prepared using methods known to the person skilled in the art of organic synthesis. They may be especially prepared according to a process starting from a compound of formula (XIII) 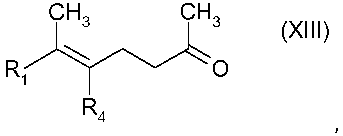 wherein R₁ signifies methyl, ethyl or n-propyl, and R₄ signifies hydrogen or methyl, and whereby
ia) the compound of formula (XIII) is hydrogenated at the C=C double bond to a compound of formula (XIV) with R₁ as defined above and with R₅ = - H, 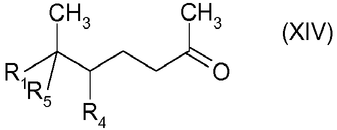
ii) the compound of formula (XIV) obtained in step ia) with R₅ = - H is then ethinylated to a compound of formula (XV), and 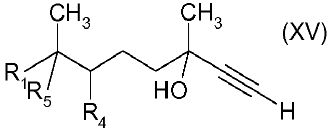
iii) the compound of formula (XV) is acylated to a compound of formula (XVI) with R₁ and R₅ as defined above, and 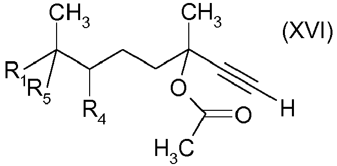
(iv) the compound of formula (XV) obtained in step ii) or the compound of formula (XVI) obtained in step iii) are hydrogenated to a compound of formula (I).

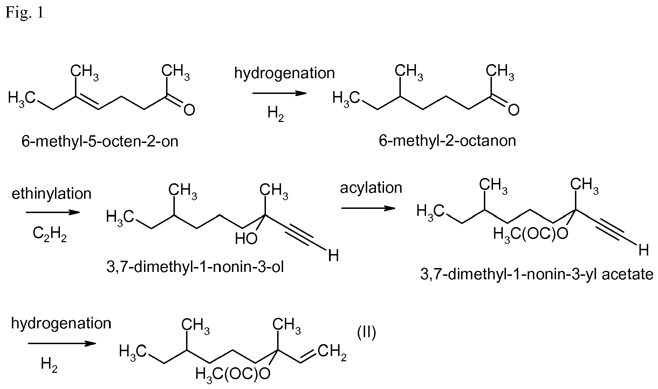

Such a process is also part of the present invention, as well as a process for the manufacture of a compound of formula (II), (V) and (VII) as shown in Figures 1-3.

Furthermore the present invention relates to the following compounds of formulae (II), (V) and (VII) which are novel compounds:

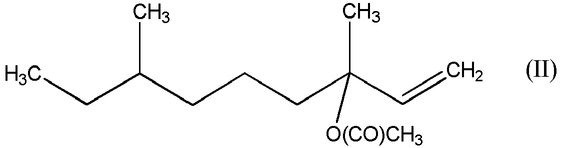

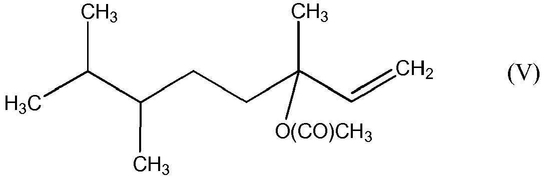

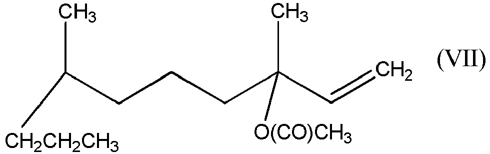

These novel compounds may be produced (manufactured, synthesized) as disclosed below. The present invention is also directed to their synthesis.

For example compound of formula (II) can be manufactured as shown in Fig. 1 starting from 6-methyl-5-octen-2-on, which is selectively hydrogenated to 6-methyl-2-octanon. 6-Methyl-2-octanon is ethinylated to 3,7-dimethyl-1-nonin-3-ol. After transformation of the hydroxy group to an acetyloxy group the thus obtained 3,7-dimethyl-1-nonin-3-yl acetate is hydrogenated in the presence of a Lindlar catalyst to the compound of formula (II).

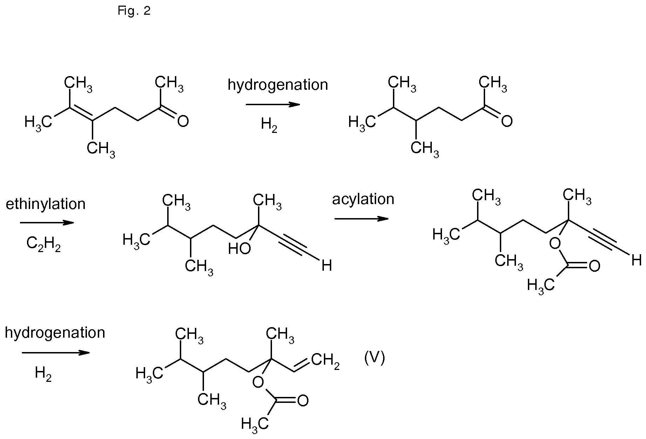

Compound of formula (V) may be manufactured starting from 5,6-dimethyl-5-hepten-2-one which is first selectively hydrogenated at the double bond, then ethinylated and after "protecting" the hydroxy group as acetyloxy group selectively hydrogenated to the compound of formula (V) in the presence of a Lindlar catalyst as shown in Fig. 2.

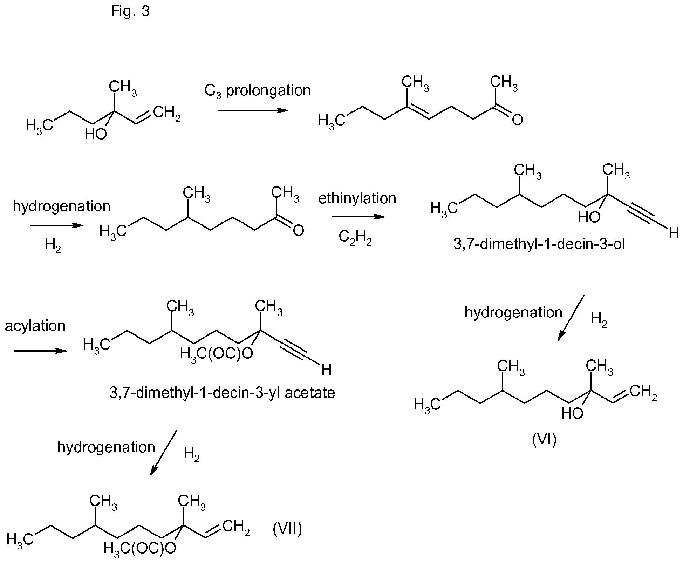

The compound of formula (VII) may be manufactured starting from 3-methyl-1-hexen-3-ol as shown in Fig. 3. 3-methyl-1-hexen-3-ol is reacted with IPM (isopropenyl methyl ether) to 6-methyl-5-nonen-2-on. After selective hydrogenation of the double bond an ethinylation takes place leading to 3,7-dimethyl-1-decin-3-ol. Acylation and hydrogenation of the triple to the double bond leads to the compound of formula (VII).

The ethinylations may be carried out as e.g. disclosed in EP-A 1 432 667 and EP-A 1 532 092.

The invention is now further illustrated in the following non-limiting examples.

### Examples

All compounds were evaluated by a panel of four persons for their intensity whereby a range of 1 to 10 was used (1 = very low intensity; 10 = very high intensity). Furthermore these four persons also described the odor of the compounds. The tenancy was evaluated by one person after 3, 6, 8, 24, 48, 72 and 96 hours. For such evaluations a piece of paper was immersed in each single liquid compound as such.

### Example 1: Manufacture of the compound of formula II and its olfactory properties

### a) Manufacture of 6-methyl-2-octanon by hydrogenation of 6-methyl-5-octen-2-on

900.0 g of 6-methyl-5-octen-2-on and 1.00 g of 5% Pd on carbon (Pd/C) are put in an autoclave under nitrogen and heated to a temperature of 60°C under stirring. Then the nitrogen is exchanged by hydrogen and put to an absolute pressure of 2 bar. After the calculated amount of hydrogen has been consumed the reaction mixture is filtered and distilled (5 mbar, 85°C) to obtain 6-methyl-2-octanon.

### b) Manufacture of 3,7-dimethyl-1-nonin-3-ol by ethinylation of 6-methyl-2-octanon

1020.0 g of 6-methyl-2-octanon are put in an autoclave under nitrogen and cooled down to a temperature of 15°C. 1920.0 g of ammonia (NH₃) are added. The reaction mixture is cooled again to 15°C. Then acetylene (C₂H₂) is added. The reaction mixture is cooled again to 15°C. Then 19.80 g of a 40 weight-% aqueous potassium hydroxide (KOH) solution are added continuously. After the end of the reaction the reaction mixture is neutralized with acetic acid, extracted with water and the solvent removed. The resulting raw product is then distilled to obtain 3,7-dimethyl-1-nonin-3-ol.

### c) Manufacture of 3,7-dimethyl-1-nonin-3-yl acetate

440.23 g of 3,7-dimethyl-1-nonin-3-ol and 0.45 g ofp-toluene sulfonic acid in water are mixed and heated up to a temperature of 40°C. 345.40 g of acetic acid anhydride are added within 2 hours. After ca. 20 hours the reaction mixture is cooled down and distilled to obtain 3,7-dimethyl-1-nonin-3-yl acetate.

### d) Manufacture of compound of formula (II) by hydrogenation of 3,7-dimethyl-1 -nonin-3-yl acetate

366.95 g of 3,7-dimethyl-1-nonin-3-yl acetate, 7.5 g of Lindlar catalyst (5% Pd + 3.5% Pb on CaCO₃), 0.04 g of ethylenedithiodiethanol and 0.02 g of zinc acetate are put in an autoclave and heated under nitrogen to a temperature of 45°C. Nitrogen is exchanged by hydrogen (H2) and the reaction mixture put at an absolute pressure of 2 bar. After the calculated amount of hydrogen has been consumed the reaction mixture is filtered and distilled (1 mbar, 120°C) to obtain 3,7-dimethyl-1-nonene-3-yl acetate (= compound of formula II).

### e) Olfactory properties

Odor description: pleasantly good; fresh green wood; nut note; cosmetic powder; sweet woodruff; lemonade powder.
Intensity: 5.
Tenancy: 3-6 hours.

### Example 2: Manufacture of the compound of formula V and its olfactory properties

### a) Manufacture of 5,6-dimethyl-2-heptanon by hydrogenation of 5,6-dimethyl-5-hepten-2-on

1400.0 g of 5,6-dimethyl-5-hepten-2-on and 3.3 g of 5% Pd/C are put in an autoclave and heated under nitrogen to a temperature of 60°C. Nitrogen is exchanged by hydrogen (H2) and the reaction mixture put at an absolute pressure of 2 bar. After the calculated amount of hydrogen has been consumed the reaction mixture is filtered and distilled (5 mbar, 85°C) to obtain 5,6-dimethyl-2-heptanon.

### b) Manufacture of 3,6,7-trimethyl-1-octin-3-ol by ethinylation of 5,6-dimethyl-2-heptanon

1272.0 g of 5,6-dimethyl-2-heptanon are put in an autoclave under nitrogen and cooled down to a temperature of 15°C. 2557.0 g of ammonia (NH₃) are added. The reaction mixture is cooled again to 15°C. Then acetylene (C₂H₂) is added. The reaction mixture is cooled again to 15°C. Then 26.4 g of a 40 weight-% aqueous potassium hydroxide (KOH) solution are added continuously. After the end of the reaction the reaction mixture is neutralized with acetic acid, extracted with water and the solvent removed. The resulting raw product is then distilled to obtain 3,6,7-trimethyl-1-octin-3-ol.

### c) Manufacture of 3,6,7-trimethyl-1-octin-3-yl acetate

757.4 g of 3,6,7-trimethyl-1-octin-3-ol and 0.73 g ofp-toluene sulfonic acid in water are mixed and heated up to a temperature of 40°C. 555.1 g of acetic acid anhydride are added within 2 hours. After ca. 20 hours the reaction mixture is cooled down and distilled to obtain 3,6,7-trimethyl-1-octin-3-yl acetate.

### d) Manufacture of the compound of formula (V) by hydrogenation of 3,6,7-trimethyl-1-octin-3-yl acetate

667.0 g of 3,6,7-trimethyl-1-octin-3-yl acetate, 11.0 g of Lindlar catalyst (5% Pd + 3.5% Pb on CaCO₃) and 0.03 g of ethylenedithiodiethanol are put in an autoclave and heated under nitrogen to a temperature of 45°C. Nitrogen is exchanged by hydrogen (H2) and the reaction mixture put at an absolute pressure of 2 bar. After the calculated amount of hydrogen has been consumed the reaction mixture is filtered and distilled (2 mbar, 115°C) to obtain 3,6,7-trimethyl-1-octen-3-yl acetate (= compound of formula V).

### e) Olfactory properties

Odor description: dry leaves; fresh washed clothes.
Intensity: 2.
Tenancy: 3-6 hours.

### Example 3: Manufacture of the compound of formula VII and its olfactory properties

### a) Manufacture of 6-methyl-2-nonanon by hydrogenation of 6-methyl-5-nonen-2-on

900.0 g of 6-methyl-5-nonen-2-on and 1.6 g of 5% Pd/C are put in an autoclave and heated under nitrogen to a temperature of 60°C. Nitrogen is exchanged by hydrogen (H2) and the reaction mixture put at an absolute pressure of 2 bar. After the calculated amount of hydrogen has been consumed the reaction mixture is filtered and distilled (10 mbar, 120°C) to obtain 6-methyl-2-nonanon.

### b) Manufacture of 3,7-dimethyl-1-decin-3-ol by ethinylation of 6-methyl-2-nonanon

760.0 g of 6-methyl-2-nonanon are put in an autoclave under nitrogen and cooled down to a temperature of 15°C. 1305.0 g of ammonia (NH₃) are added. The reaction mixture is cooled again to 15°C. Then acetylene (C₂H₂) is added. The reaction mixture is cooled again to 15°C. Then 14.0 g of a 40 weight-% aqueous potassium hydroxide (KOH) solution are added continuously. After the end of the reaction the reaction mixture is neutralized with acetic acid, extracted with water and the solvent removed. The resulting raw product is then distilled to obtain 3,7-dimethyl-1-decin-3-ol.

### c) Manufacture of 3,7-dimethyl-1-decin-3-yl acetate

472.0 g of 3,7-dimethyl-1-decin-3-ol and 0.42 g of p-toluene sulfonic acid in water are mixed and heated up to a temperature of 40°C. 317.4 g of acetic acid anhydride are added within 2 hours. After ca. 20 hours the reaction mixture is cooled down and distilled to obtain 3,7-dimethyl-1-decin-3-yl acetate.

### d) Manufacture of the compound of formula (VII) by hydrogenation of 3,7-dimethyl-1-decin-3-yl acetate

310.0 g of 3,7-dimethyl-1-decin-3-yl acetate, 2.4 g of Lindlar catalyst (5% Pd + 3.5% Pb on CaCO₃), 0.01 g of ethylenedithiodiethanol and 0.11 g of zinc acetate are put in an autoclave and heated under nitrogen to a temperature of 45°C. Nitrogen is exchanged by hydrogen (H2) and the reaction mixture put at an absolute pressure of 2 bar. After the calculated amount of hydrogen has been consumed the reaction mixture is filtered and distilled (2 mbar, 140°C) to obtain 3,7-dimethyl-1-decen-3-yl acetate (= compound of formula VII).

### e) Olfactory properties

Odor description: vinegar; green; herb; grass.
Intensity: 3.5.
Tenancy: 8-24 hours.

## Claims

1. Use of a compound of formula (I) 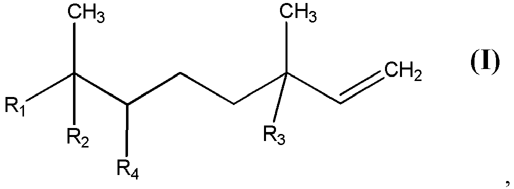 wherein
R₁ signifies - CH₃, -CH₂CH₃ or -CH₂CH₂CH₃, and
R₂ signifies -H, and
R₃ signifies -O(CO)CH₃, and
R₄ signifies -H or -CH₃;
with the proviso that R¹ is not methyl, if R² and R⁴ signify hydrogen;
as flavor and fragrance material.

2. Use according to claim 1, wherein at least one compound of formulae (Ia) - (Ic), (Ie), (If), (Ih) and (Ii) 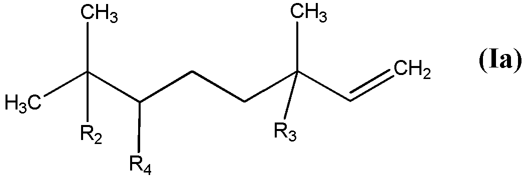 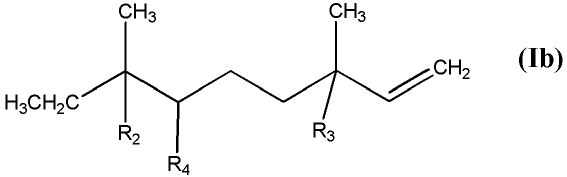 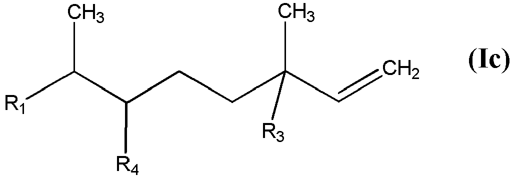 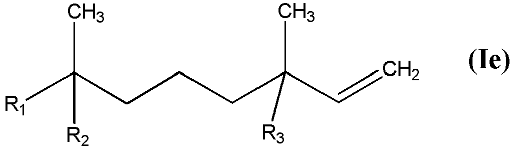 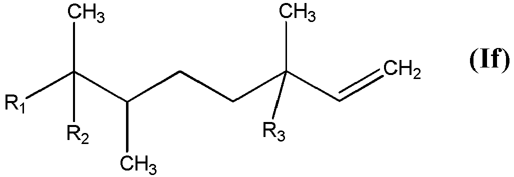 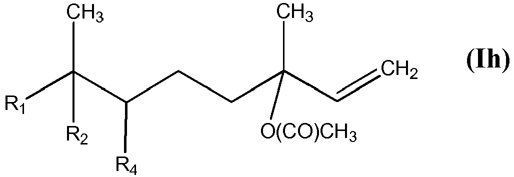 and 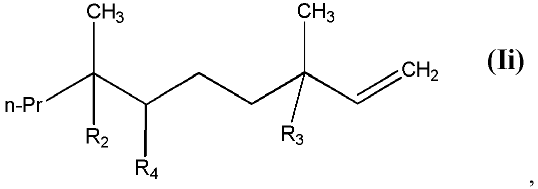 wherein R₁, R₂, R₃ and R₄ have the meanings as defined in Claim 1,
is used.

3. A flavor and fragrance formulation comprising
(i) at least one compound of formula (I) 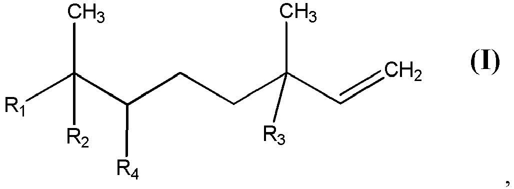 wherein
R₁ signifies - CH₃, -CH₂CH₃ or -CH₂CH₂CH₃, and
R₂ signifies -H, and
R₃ signifies -O(CO)CH₃, and
R₄ signifies -H or -CH₃;
with the proviso that R¹ is **not** methyl, if R² **and** R⁴ signify hydrogen.

4. Flavor and fragrance formulation according to claim 3 comprising 0.0001 - 10 wt-%, related to the total weight of the flavor and fragrance formulation, of at least one compound of formula (I).

5. Flavor and fragrance formulation according to claim 3 and 4, wherein the flavor and fragrance formulation is solid, gel-like or liquid.

6. Flavor and fragrance formulation according to any of claims 3-5, wherein the flavor and fragrance formulation is a perfume, air care product, household product, laundry product, body care product or cosmetic product.

7. A method of improving, enhancing or modifying a flavor and fragrance formulation by means of addition thereto an olfactory acceptable amount of at least one compound of formula (I) 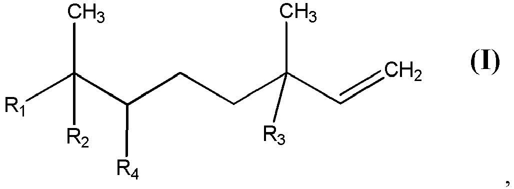 wherein
R₁ signifies - CH₃, -CH₂CH₃ or -CH₂CH₂CH₃, and
R₂ signifies -H, and
R₃ signifies -O(CO)CH₃, and
R₄ signifies -H or -CH₃;
with the proviso that R¹ is **not** methyl, if R² **and** R⁴ signify hydrogen.

8. A compound of formula (II), (V), and (VII)

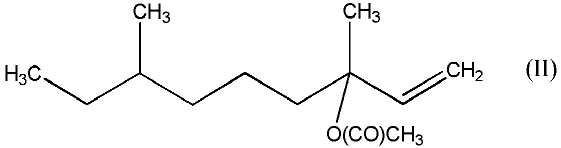

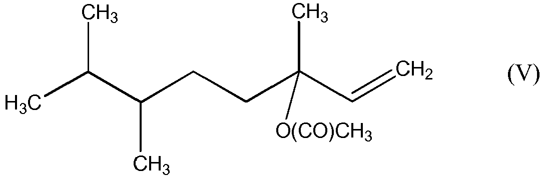

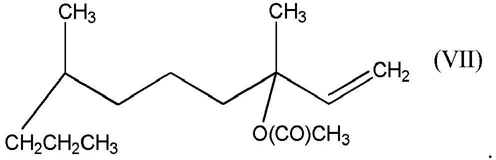

9. A process for the manufacture of a compound of formula (I) 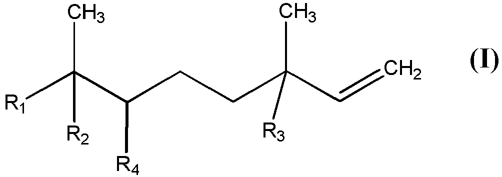 as defined in claim 1 starting from a compound of formula (XIII) 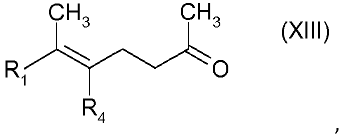 wherein R₁ signifies methyl, ethyl or n-propyl, and R₄ signifies hydrogen or methyl, and whereby
ia) the compound of formula (XIII) is hydrogenated at the C=C double bond to a compound of formula (XIV) with R₁ as defined above and with R₅ = - H, 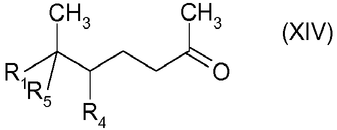 and
ii) the compound of formula (XIV) obtained in step ia) with R₅ = - H is then ethinylated to a compound of formula (XV), and 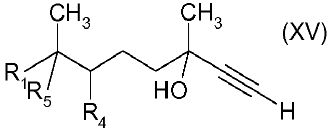
iii) the compound of formula (XV) is acylated to a compound of formula (XVI) with R₁ and R₅ as defined above, 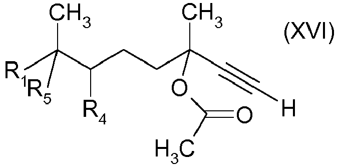 and
(iv) the compound of formula (XV) obtained in step ii) or the compound of formula (XVI) obtained in step iii) are hydrogenated to a compound of formula (I).

10. A process for the manufacture of a compound of formula (II) comprising the following steps:
- hydrogenation of 6-methyl-5-octen-2-on with hydrogen to obtain 6-methyl-2-octanon;
- ethinylation of the thus obtained 6-methyl-2-octanon with acetylene to obtain 3,7-dimethyl-1-nonin-3-ol;
- acetylation of the thus obtained 3,7-dimethyl-1-nonin-3-ol to obtain 3,7-dimethyl-1-nonin-3-yl acetate;
- hydrogenation of the thus obtained 3,7-dimethyl-1-nonin-3-yl acetate with hydrogen to obtain the compound of formula (II).

11. A process for the manufacture of a compound of formula (V) comprising the following steps:
- hydrogenation of 5,6-dimethyl-5-hepten-2-on with hydrogen to obtain 5,6-dimethyl-heptan-2-on;
- ethinylation of the thus obtained 5,6-dimethyl-heptan-2-on with acetylene to obtain 3,6,7-trimethyl-1-octin-3-ol;
- acetylation of the thus obtained 3,6,7-trimethyl-1-octin-3-ol to obtain 3,6,7-trimethyl-1-octin-3-yl acetate;
- hydrogenation of the thus obtained 3,6,7-trimethyl-1-octin-3-yl acetate with hydrogen to obtain the compound of formula (V).

12. A process for the manufacture of a compound of formula (VII) comprising the following steps:
- C3 prolongation of 3-methyl-1-hexen-3-ol to obtain 6-methyl-5-nonen-2-on;
- hydrogenation of 6-methyl-5-nonen-2-on with hydrogen to obtain 6-methyl-nonan-2-on;
- ethinylation of the thus obtained 6-methyl-nonan-2-on with acetylene to obtain 3,7-dimethyl-1-decin-3-ol;
- acetylation of the thus obtained 3,7-dimethyl-1-decin-3-ol to obtain 3,7-dimethyl-1-decin-3-yl acetate;
- hydrogenation of the thus obtained 3,7-dimethyl-1-decin-3-yl acetate with hydrogen to obtain the compound of formula (VII).

## Patentansprüche

1. Verwendung einer Verbindung der Formel (I) 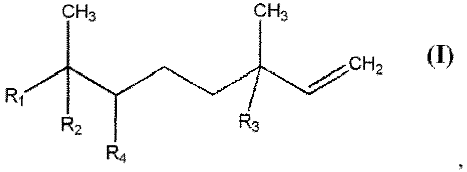 wobei
R₁ für -CH₃, -CH₂CH₃ oder -CH₂CH₂CH₃ steht und
R₂ für -H steht und
R₃ für -O(CO)CH₃ steht und
R₄ für -H oder -CH₃ steht;
mit der Maßgabe, dass R¹ nicht Methyl ist, wenn R₂ und R⁴ für Wasserstoff stehen;
als Geschmacks- und Duftstoff.

2. Verwendung nach Anspruch 1, wobei mindestens eine Verbindung der Formeln (Ia)-(Ic), (Ie), (If), (Ih) und (Ii) 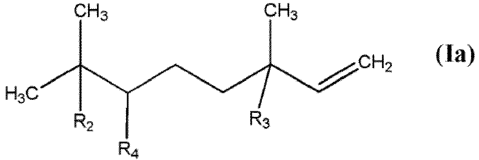 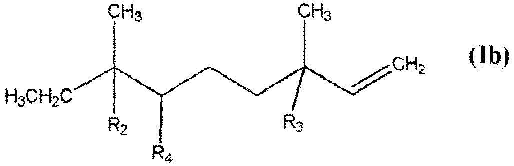 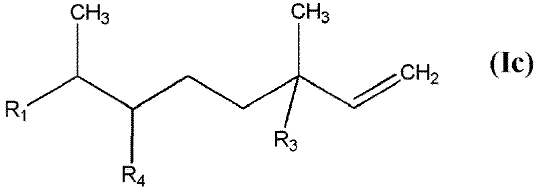 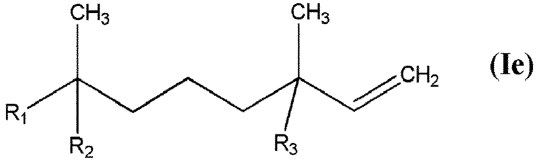 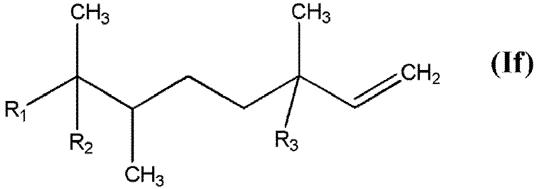 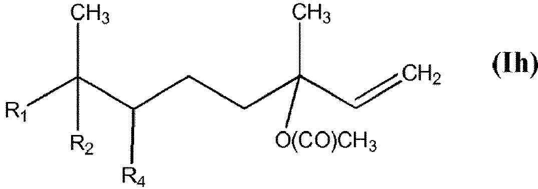 und 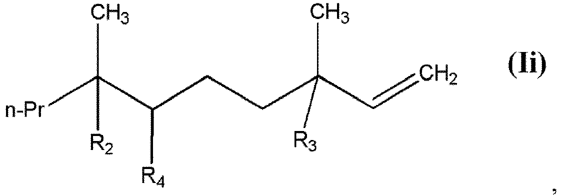 wobei R₁, R₂, R₃ und R₄ die in Anspruch 1 angegebenen Bedeutungen haben,
verwendet wird.

3. Geschmacks- und Duftstoffformulierung, umfassend
(i) mindestens eine Verbindung der Formel (I) 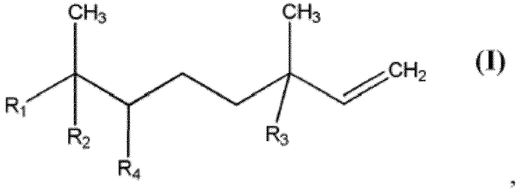 wobei
R₁ für -CH₃, -CH₂CH₃ oder -CH₂CH₂CH₃ steht und
R₂ für -H steht und
R₃ für -O(CO)CH₃ steht und
R₄ für -H oder -CH₃ steht;
mit der Maßgabe, dass R¹ nicht Methyl ist, wenn R² und R⁴ für Wasserstoff stehen.

4. Geschmacks- und Duftstoffformulierung nach Anspruch 3, umfassend 0,0001-10 Gew.-%, bezogen auf das Gesamtgewicht der Geschmacks- und Duftstoffformulierung, mindestens einer Verbindung der Formel (I).

5. Geschmacks- und Duftstoffformulierung nach Anspruch 3 oder 4, wobei die Geschmacks- und Duftstoffformulierung fest, gelartig oder flüssig ist.

6. Geschmacks- und Duftstoffformulierung nach einem der Ansprüche 3-5, wobei es sich bei der Geschmacks- und Duftstoffformulierung um ein Parfüm, ein Luftpflegeprodukt, ein Haushaltsprodukt, ein Waschmittelprodukt, ein Körperpflegeprodukt oder ein Kosmetikprodukt handelt.

7. Verfahren zur Verbesserung, Verstärkung oder Modifizierung einer Geschmacks- und Duftstoffformulierung durch Versetzen mit einer olfaktorisch unbedenklichen Menge mindestens einer Verbindung der Formel (I) 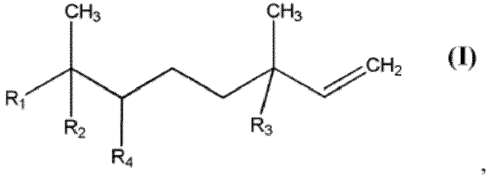 wobei
R₁ für -CH₃, -CH₂CH₃ oder -CH₂CH₂CH₃ steht und
R₂ für -H steht und
R₃ für -O(CO)CH₃ steht und
R₄ für -H oder -CH₃ steht;
mit der Maßgabe, dass R¹ nicht Methyl ist, wenn R² und R⁴ für Wasserstoff stehen.

8. Verbindung der Formel (II), (V) und (VII)

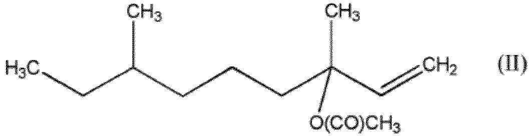

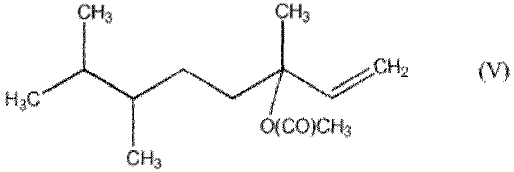

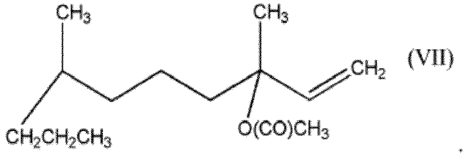

9. Verfahren zur Herstellung einer Verbindung der Formel (I) 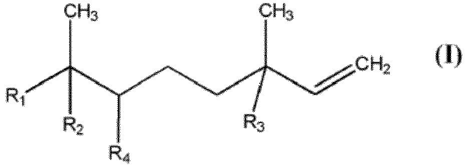 gemäß Anspruch 1 ausgehend von einer Verbindung der Formel (XIII) 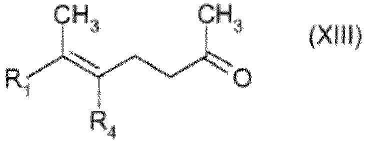 wobei
R₁ für Methyl, Ethyl oder n-Propyl steht und R₄ für Wasserstoff oder Methyl steht, und wobei
ia) die Verbindung der Formel (XIII) an der C=C-Doppelbindung zu einer Verbindung der Formel (XIV) hydriert wird, wobei R₁ wie oben definiert ist und wobei R₅ = -H, 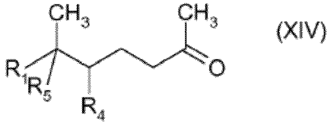 und
ii) die in Schritt ia) erhaltene Verbindung der Formel (XIV) mit R₅ = -H dann zu einer Verbindung der Formel (XV) ethinyliert wird und 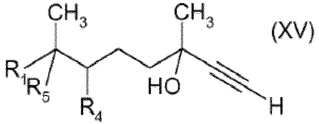
iii) die Verbindung der Formel (XV) zu einer Verbindung der Formel (XVI) acyliert wird, wobei R₁ und R₅ wie oben definiert sind, 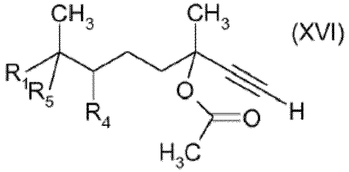 und
(iv) die in Schritt ii) erhaltene Verbindung der Formel (XV) oder die in Schritt iii) erhaltene Verbindung der Formel (XVI) zu einer Verbindung der Formel (I) hydriert wird.

10. Verfahren zur Herstellung einer Verbindung der Formel (II), das die folgenden Schritte umfasst:
- Hydrierung von 6-Methyl-5-octen-2-on mit Wasserstoff zu 6-Methyl-2-octanon;
- Ethinylierung des so erhaltenen 6-Methyl-2-octanons mit Acetylen zu 3,7-Dimethyl-1-nonin-3-ol;
- Acetylierung des so erhaltenen 3,7-Dimethyl-1-nonin-3-ols zu Essigsäure-3,7-dimethyl-1-nonin-3-ylester;
- Hydrierung des so erhaltenen Essigsäure-3,7-dimethyl-1-nonin-3-ylesters mit Wasserstoff zu der Verbindung der Formel (II).

11. Verfahren zur Herstellung einer Verbindung der Formel (V), das die folgenden Schritte umfasst:
- Hydrierung von 5,6-Dimethyl-5-hepten-2-on mit Wasserstoff zu 5,6-Dimethylheptan-2-on;
- Ethinylierung des so erhaltenen 5,6-Dimethyl-heptan-2-ons mit Acetylen zu 3,6,7-Trimethyl-1-octin-3-ol;
- Acetylierung des so erhaltenen 3,6,7-Trimethyl-1-octin-3-ols zu Essigsäure-3,6,7-trimethyl-1-octin-3-ylester;
- Hydrierung des so erhaltenen Essigsäure-3,6,7-trimethyl-1-octin-3-ylesters mit Wasserstoff zu der Verbindung der Formel (V).

12. Verfahren zur Herstellung einer Verbindung der Formel (VII), das die folgenden Schritte umfasst:
- C3-Verlängerung von 3-Methyl-1-hexen-3-ol zu 6-Methyl-5-nonen-2-on;
- Hydrierung von 6-Methyl-5-nonen-2-on mit Wasserstoff zu 6-Methylnonan-2-on;
- Ethinylierung des so erhaltenen 6-Methylnonan-2-ons mit Acetylen zu 3,7-Dimethyl-1-decin-3-ol;
- Acetylierung des so erhaltenen 3,7-Dimethyl-1-decin-3-ols zu Essigsäure-3,7-dimethyl-1-decin-3-ylester;
- Hydrierung des so erhaltenen Essigsäure-3,7-dimethyl-1-decin-3-ylesters mit Wasserstoff zu der Verbindung der Formel (VII).

## Revendications

1. Utilisation d'un composé de formule (I) 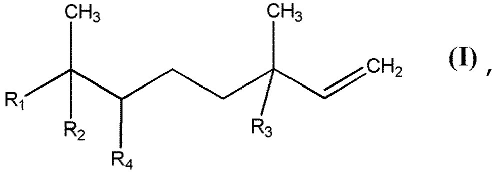 où
R₁ désigne -CH₃, -CH₂CH₃ ou -CH₂CH₂CH₃ et
R₂ désigne -H et
R₃ désigne -O(CO)CH₃ et
R₄ désigne -H ou -CH₃ ;
à condition que R₁ ne soit pas un groupe méthyle si R₂ et R₄ désignent des atomes d'hydrogène ;
en tant que substance d'arôme et de fragrance.

2. Utilisation selon la revendication 1, dans laquelle au moins un composé représenté par les formules (Ia)-(Ic), (Ie), (If), (Ih) et (Ii) 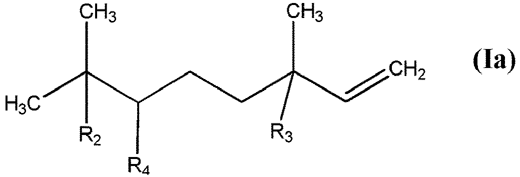 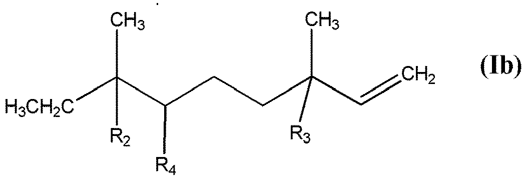 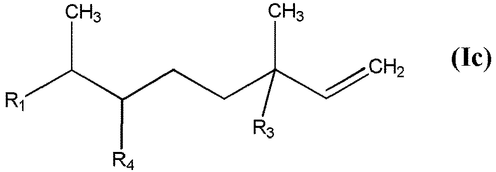 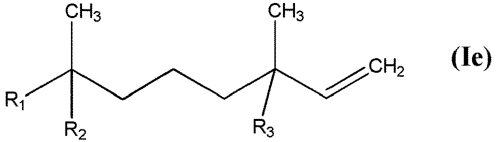 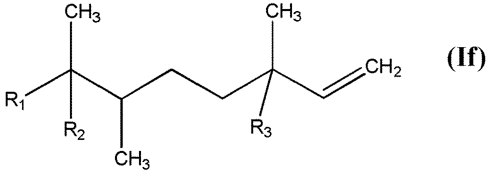 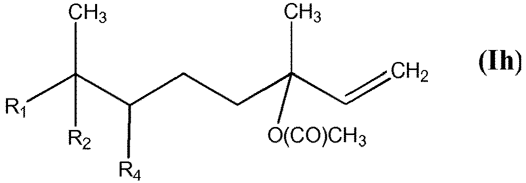 et 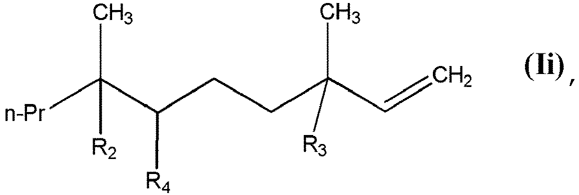 où R₁, R₂, R₃ et R₄ ont les significations telles que définies dans la revendication 1,
est utilisé.

3. Formulation d'arôme et de fragrance comprenant
(i) au moins un composé de formule (I) 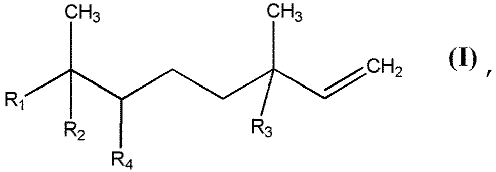 où
R₁ désigne -CH₃, -CH₂CH₃ ou -CH₂CH₂CH₃ et
R₂ désigne -H et
R₃ désigne -O(CO)CH₃ et
R₄ désigne -H ou -CH₃ ;
à condition que R₁ ne soit **pas** un groupe méthyle si R₂ **et** R₄ désignent des atomes d'hydrogène.

4. Formulation d'arôme et de fragrance selon la revendication 3 comprenant 0,0001-10 % en poids, par rapport au poids total de la formulation d'arôme et de fragrance, d'au moins un composé de formule (I).

5. Formulation d'arôme et de fragrance selon les revendications 3 et 4, la formulation d'arôme et de fragrance étant solide, sous forme de gel ou liquide.

6. Formulation d'arôme et de fragrance selon l'une quelconque des revendications 3-5, la formulation d'arôme et de fragrance étant un parfum, un produit d'assainissement de l'air, un produit ménager, un produit pour la lessive, un produit de soins corporels ou un produit cosmétique.

7. Procédé d'amélioration, d'amplification ou de modification d'une formulation d'arôme et de fragrance au moyen de l'ajour à celle-ci d'une quantité olfactivement acceptable d'au moins un composé de formule (I) 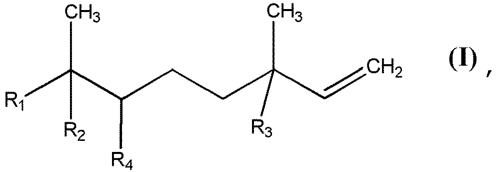 où
R₁ désigne -CH₃, -CH₂CH₃ ou -CH₂CH₂CH₃ et
R₂ désigne -H et
R₃ désigne -O(CO)CH₃ et
R₄ désigne -H ou -CH₃ ;
à condition que R₁ ne soit **pas** un groupe méthyle si R₂ **et** R₄ désignent des atomes d'hydrogène.

8. Composé de formule (II), (V) et (VII)

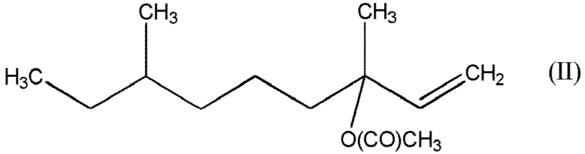

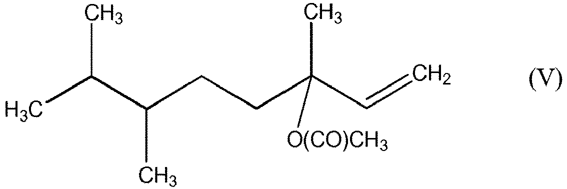

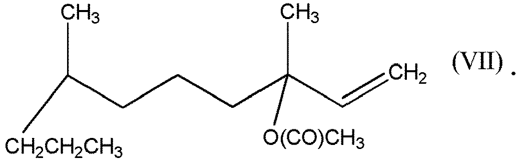

9. Procédé pour la fabrication d'un composé de formule (I) 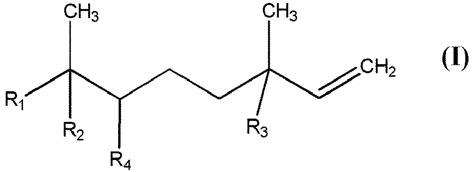 tel que défini dans la revendication 1 à partir d'un composé de formule (XIII) 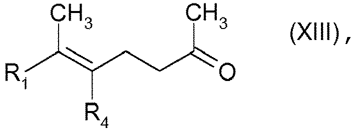 où R₁ désigne un groupe méthyle, éthyle ou n-propyle et R₄ désigne un atome d'hydrogène ou un groupe méthyle, et par lequel
ia) le composé de formule (XIII) est hydrogéné au niveau de la double liaison C=C en un composé de formule (XIV), R₁ étant tel que défini ci-dessus et R₅ étant -H, 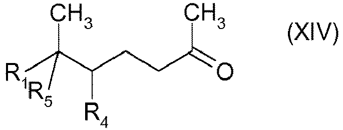 et
ii) le composé de formule (XIV) obtenu dans l'étape ia), R₅ étant -H, est ensuite éthynylé en un composé de formule (XV) 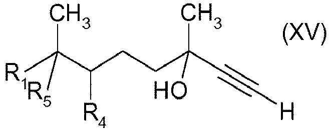 et
iii) le composé de formule (XV) est acylé en un composé de formule (XVI), R₁ et R₅ étant tels que définis ci-dessus, 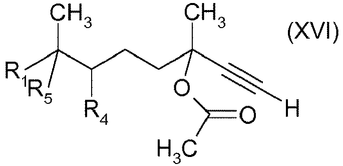 et
iv) le composé de formule (XV) obtenu dans l'étape ii) ou le composé de formule (XVI) obtenu dans l'étape iii) sont hydrogénés en un composé de formule (I).

10. Procédé pour la fabrication d'un composé de formule (II) comprenant les étapes suivantes :
- l'hydrogénation de 6-méthyloct-5-én-2-one avec de l'hydrogène pour obtenir de la 6-méthyloctan-2-one ;
- l'éthynylation de la 6-méthyloctan-2-one ainsi obtenue avec de l'acétylène pour obtenir du 3,7-diméthylnon-1-yn-3-ol ;
- l'acétylation du 3,7-diméthylnon-1-yn-3-ol ainsi obtenu pour obtenir de l'acétate de 3,7-diméthylnon-1-yn-3-yle ;
- l'hydrogénation de l'acétate de 3,7-diméthylnon-1-yn-3-yle ainsi obtenu avec de l'hydrogène pour obtenir le composé de formule (II).

11. Procédé pour la fabrication d'un composé de formule (V) comprenant les étapes suivantes :
- l'hydrogénation de 5,6-diméthylhept-5-én-2-one avec de l'hydrogène pour obtenir de la 5,6-diméthylheptan-2-one ;
- l'éthynylation de la 5,6-diméthylheptan-2-one ainsi obtenue avec de l'acétylène pour obtenir du 3,6,7-triméthyloct-1-yn-3-ol;
- l'acétylation du 3,6,7-triméthyloct-1-yn-3-ol ainsi obtenu pour obtenir de l'acétate de 3,6,7-triméthyloct-1-yn-3-yle;
- l'hydrogénation de l'acétate de 3,6,7-triméthyloct-1-yn-3-yle ainsi obtenu avec de l'hydrogène pour obtenir le composé de formule (V).

12. Procédé pour la fabrication d'un composé de formule (VII) comprenant les étapes suivantes :
- la prolongation en C3 de 3-méthylhex-1-én-3-ol pour obtenir de la 6-méthylnon-5-én-2-one ;
- l'hydrogénation de 6-méthylnon-5-én-2-one avec de l'hydrogène pour obtenir de la 6-méthylnonan-2-one ;
- l'éthynylation de la 6-méthylnonan-2-one ainsi obtenue avec de l'acétylène pour obtenir du 3,7-diméthyldéc-1-yn-3-ol;
- l'acétylation du 3,7-diméthyldéc-1-yn-3-ol ainsi obtenu pour obtenir de l'acétate de 3,7-diméthyldéc-1-yn-3-yle;
- l'hydrogénation de l'acétate de 3,7-diméthyldéc-1-yn-3-yle ainsi obtenu avec de l'hydrogène pour obtenir le composé de formule (VII).
